Europäisches Patentamt

European Patent Office

Office européen des brevets

(19)

(11) Veröffentlichungsnummer: **0 288 734 B1**

(12) **EUROPÄISCHE PATENTSCHRIFT**

(45) Veröffentlichungstag der Patentschrift: **04.12.91**

(21) Anmeldenummer: **88104580.1**

(22) Anmeldetag: **22.03.88**

�IntCl.⑤: **A61M 36/00, A61L 15/16**

(54) **Wirkstoffhaltiges Pflaster zur kontrollierten Verabreichung von Wirkstoffen an die Haut.**

(30) Priorität: **28.04.87 DE 3714140**

(43) Veröffentlichungstag der Anmeldung:
**02.11.88 Patentblatt 88/44**

(45) Bekanntmachung des Hinweises auf die
Patenterteilung:
**04.12.91 Patentblatt 91/49**

(84) Benannte Vertragsstaaten:
**AT BE CH DE ES FR GB GR IT LI LU NL SE**

(56) Entgegenhaltungen:
**EP-A- 0 178 601**
**US-A- 4 297 995**

(73) Patentinhaber: **LTS Lohmann Therapie-**
**Systeme GmbH & Co. KG**
**Irlicherstrasse 55**
**W-5450 Neuwied 12(DE)**

(72) Erfinder: **Hoffmann, Hans Rainer, Dr.**
**Burghofstrasse 113**
**W-5450 Neuwied 22(DE)**
Erfinder: **Wolter, Karin, Dr.**
**Altwieder Strasse 46**
**W-5451 Melsbach(DE)**
Erfinder: **Simon, Günter**
**Tulpenweg 1**
**W-5333 Hillesheim(DE)**
Erfinder: **Barth, Peter, Dr.**
**Johann-Gottfried-Herder-Strasse 6**
**W-5450 Neuwied 12(DE)**

(74) Vertreter: **Neidl-Stippler, Cornelia, Dr.**
**Rauchstrasse 2**
**W-8000 München 80(DE)**

## Beschreibung

Die Erfindung betrifft ein wirkstoffhaltiges Pflaster zur kontrollierten Verabreichung von Wirkstoffen an die Haut, das eine Rückseite und eine Hautseite besitzt, mit einer Rückschicht, einem im wesentlichen senkrecht zur Hautkontaktfläche des Pflasters geteilten, einen oder mehrere Wirkstoffe aufweisenden Wirkstoffreservoir, einer Haftklebeeinrichtung auf der Hautseite sowie ggf. einer vor Applikation des Pflasters ablösbaren Abdeckschicht, wobei die Wirkstoffabgabefläche des Pflasters um eine vorherbestimmte Fläche durch Entfernung eines Teils des Wirkstoffreservoirs verkleinerbar ist sowie dessen Verwendung und ein Verfahren zur kontrollierten Verabreichung von einem oder mehreren Wirkstoffen an die Haut mittels eines Pflasters, jedoch ausgenommen für die therapeutische Behandlung des menschlichen oder tierischen Körpers oder human- oder veterinärmedizinische Diagnostizierverfahren.

Die Erfindung bezieht sich insbesondere auf solche Pflaster, wie sie als transdermale therapeutische Systeme zur gesteuerten Verabreichung medizinischer Wirkstoffe oder auch kosmetisch wirksamer Substanzen an die menschliche oder tierische Haut eingesetzt werden. Ein therapeutisches System ist eine arzneistoff- bzw. wirkstoffenthaltende Vorrichtung bzw. Darreichungsform, die einen oder mehrere Wirkstoffe in einer vorherbestimmten Rate kontinuierlich über einen festgelegten Zeitraum an einem festgelegten Anwendungsort abgibt.

Diese pflasterartigen Systeme sind therapeutische Präzisionsinstrumente, deren Konstruktion außergewöhnliche Maßnahmen erfordert, um die kontinuierliche Wirkstofffreisetzung sicherzustellen.

Pflasterartige therapeutische Systeme sind schon für die verschiedensten Anwendungen entwickelt worden, wobei neben topischer auch systemische Wirkung erzielt werden kann. Die Vielfalt der auf diese Weise applizierbaren Wirkstoffe und ihre unterschiedlichen chemischen, physikalischen und pharmakologischen Eigenschaften machen es unmöglich, mit nur einem System alle therapeutischen Problemstellungen abzudecken.

Es sind schon zahlreiche pflasterartige therapeutische Systeme zur Verabreichung von medizinischen Wirkstoffen an die Haut bekannt geworden, wobei eine Zusammenstellung bspw. in Klaus Heilmann "Therapeutische Systeme", Ferdinand Enke Verlag, Stuttgart, 1977, zu finden ist. In nicht allen Fällen konnte durch die Systeme nach dem Stand der Technik eine völlig befriedigende Wirkung erzielt werden.

Beim üblichen Aufbau eines transdermalen pflasterartigen therapeutischen Systems liegt ein Wirkstoffreservoir, in dem der Wirkstoff in fester, flüssiger oder gelöster Form anwesend ist und eine druckempfindliche Adhäsionsschicht (Haftkleber), durch die das System eng mit der Haut verbunden ist, vor. Es ist wichtig, daß ein vollflächiger Kontakt der Wirkstoffabgabeflächen mit der Haut ständig während der Applikation des pflasterartige Systems gewährleistet ist, um die Wirkstoffabgabekinetik sicherzustellen, wobei dies nicht nur durch eine ununterbrochene Adhäsionsschicht, sondern auch durch abgegrenzte Klebebereiche in der Hautkontaktschicht des Pflaster erreicht werden kann.

Die bisherigen pflasterartigen therapeutischen Systeme erlauben wohl die gleichmäßige oder kontinuierlich abnehmende Applikation eines Wirkstoffs über einen vorherbestimmten Zeitraum, nicht jedoch, eine bestimmte Wirkstoffabgabekinetik, wie eine gezielte Reduktion der Wirkstoffabgabe nach einem vorherbestimmten Zeitraum, wie eine abgestufte Abgabemenge Wirkstoff pro Zeiteinheit einzustellen.

Diese Maßnahme wird bspw. dann erforderlich, wenn die Wirkstoffdosis aus therapeutischen Gründen während der Applikationsdauer gezielt herabgesetzt werden muß. Das gleiche Problem ergibt sich, wenn bei Applikation eines Kombinationspräparates ein Wirkstoff der Kombination nach einem vorherbestimmten Zeitraum abgesetzt werden muß. In diesen Fällen kann man sich prinzipiell zwar dadurch helfen, indem zwei transdermale Pflaster eingesetzt werden, von denen das eine zur gegebenen Zeit abgezogen wird. Dies bringt für den Patienten, insbesondere ältere Patienten große Probleme mit sich. So wird leicht das Anbringen des zweiten Pflasters vergessen. Bei einer Kombination von zwei Pflastern mit verschiedenen Wirkstoffabgabeflächen besteht weiterhin die Gefahr, daß die falsche Kombination gewählt (zwei gleiche Pflaster) oder das verkehrte Pflaster wieder abgezogen wird. Dieselben Komplikationen treten auf, wenn Pflaster mit verschiedenen Wirkstoffen kombiniert werden müssen. Hinzu kommt in diesem Falle noch die doppelte Lagerhaltung. Damit ist die Verwendung von zwei Pflastern mit sehr vielen Nachteilen behaftet, deren Vermeidung ein Ziel der Erfindung ist.

Der Vorschlag, zwei Pflaster derart miteinander zu verbinden, daß mit Hilfe einer Sollbruchlinie ein Teil davon abgelöst werden kann, verbessert zwar die Compliance, führt aber zu Schwierigkeiten bei der Handhabung. So kann der abzulösende Teil nur nach Abheben von der Haut gehandhabt werden, wobei es ungewollt zum Abheben mindestens eines Teilabschnitts des auf der Haut verbleibenden Pflasterrestes kommt, so daß dieser bei der Ablösung festgehalten werden muß. Dabei wird auf das empfindliche pflasterartige transdermale System Druck ausgeübt, der die Freisetzungsrate des Wirkstoffes negativ beeinflussen kann.

In der US-PS 4,297,995 ist ein manipulierbares Wirkstoffpflaster beschrieben, bei dem das Wirkstoffreservoir unterteilt, aber in einem einzigen Pflaster integriert ist. Die Wirkstoffreservoirteile sind in konzentrischen Ringen um ein scheibchenförmiges Mittelstück angeordnet, Die Gesamtkonstruktion des Pflasters mit einer mechanischen Befestigung des Reservoirs an der Rückschicht läßt die Veränderung der Wirkstoffabgabefläche nur vor Applikation des Pflasters zu - es kann also vom Arzt/Patienten vor Aufbringen des Pflasters ausgewählt werden, welche Dosis abgegeben werden soll, für eine Veränderung der Wirkstoffabgabe während der Applikation des Pflasters muß das Pflaster unter Unterbrechung der Therapie von der Haut abgehoben werden, was auch zu Beschädigungen und Kontaminationen der Pflasterauflagefläche führen kann. Für die damit verbundenen Probleme der Repositionierung und der dauerhaften Refixierung des Systems auf der Haut ist noch keine zufriedenstellende Lösung gefunden worden.

Es ist daher Aufgabe der Erfindung, ein verbessertes pflasterartiges therapeutisches transdermales System zu schaffen, das kompliziertere Änderungen der Wirkstoffabgabe, als bisher möglich, verwirklichen kann.

Die Aufgabe wird durch ein gattungsgemäßes Pflaster gelöst, das dadurch gekennzeichnet ist, daß mindestens einTeil des Wirkstoffreservoirs unter Verbleiben eines oder mehrerer Teile des Wirkstoffreservoirs auf der Haut ablösbar ist, wobei der auf der Haut verbleibende Teil des Wirkstoffreservoirs eine bessere Haftung an der Haut als an der Rückschicht aufweist.

Dadurch, daß das Wirkstoffreservoir erfindungsgemäß erst mals partiell ablösbar ist, wobei der nicht abzulösende Anteil des Wirkstoffreservoirs zu Haut hin eine größere Haftung als zur Rückschicht besitzt, verbleibt nach Abziehen eines vorherbestimmten Pflasterteils mit daranhaftendem Wirkstoffreservoirteil ein vorherbestimmter Wirkstoffreservoiranteil auf der Haut, der bspw. nach der erwünschten Applikationsdauer alleine abgelöst werden kann.

Vorteilhafterweise ist das Wirkstoffreservoir des erfindungsgemäßen Pflasters zweiteilig.

Bei bestimmten Therapien mit unterschiedlichen oder stark in der Konzentration schwankenden Wirkstoffgaben kann es auch vorteilhaft sein, wenn das Wirkstoffreservoir dreiteilig ist.Die Wirkstoffabgabeflächen der Wirkstoffreservoirteile können geometrisch gleich oder unterschiedlich sein. Die Wirkstoffabgabeflächen können nebeneinander angeordnet sein, oder es kann ein Wirkstoffreservoirteil ein anderes oder mehrere Wirkstoffreservoirteile, flächenmäßig betrachtet, vollständig umschließen. Die Aufteilung der Teilflächen hängt dabei von den therapeutischen Erfordernissen ab. So kann bspw. ein Wirkstoffreservoirteil ringförmig einen oder mehrere andere Wirkstoffreservoirteil(e) umschließen.

Das Abgabeflächen-Größenverhältnis eines Wirkstoffreservoirteils zu einem anderen liegt bevorzugt zwischen etwa 1:1 bis 1:10.

In allen Wirkstoffreservoirteilen kann derselbe Wirkstoff oder dieselbe Wirkstoffkombination vorliegen.

Besonders bevorzugte Präparate sind Pflaster mit folgenden Wirkstoffen, wobei sich jedoch auch beliebige andere, dem Mediziner geläufige, transdermal applizierbare Wirkstoffkombinationen verarbeiten lassen: Asthma/Bronchodilatatoren, wie z.B. Clenbuterol, Procaterol und Salbutamol und Vasodilatatoren, wie z.B. Bencyclan und Cinnarizin.

In diesen Fällen gelingt es also, mit Hilfe des erfindungsgemäßen Pflasters die Verabreichungsdosie während der Applikation gezielt und gesteuert zu reduzieren.

Die Wirkstoffreservoirteile können aber auch verschiedene Wirkstoffe oder verschiedene Wirkstoffkombinationen enthalten, so daß während der Applikation ein Wirkstoff oder eine Wirkstoffkombination abgesetzt werden kann. Als Beispiel für verschiedende Wirkstoffe in den Wirkstoff-Reservoirteilen seien genannt.

Östrogen/Gestagen (Kontrazeptiva), Dexamethason/Prednisolon (bei entzündlichen rheumatischen Muskel- und Gelenkkrankheiten), Nitroglycerin/$\beta$-Blocker (bei Herzkrankheiten) Phenytoin/Phenobarbital/Coffein (bei Epilepsie) und Amitriptylin/Chlordiazepoxid (Psychopharmaka).

Alle geeigneten Wirkstoffe gehören zu den Gruppen, die entweder topische oder systemische Wirkung entfalten.

Dabei kann in mindestens einem Wirkstoffreservoirteil ein(e) andere(r) Wirkstoff/Wirkstoffkombination als in dem anderen Wirkstoffreservoirteil(en) vorliegen.

Die Verwendung der erfindungsgemäßen Pflaster liegt in der lokalen und systemischen transdermalen Wirkstoffverabreichung in der Kosmetik.

Die Erfindung betrifft ferner auch ein Verfahren zur kontrollierten Verabreichung von einem oder mehreren Wirkstoffen an die Haut mittels eines Pflasters gemäß einem der vorangehenden Ansprüche, jedoch ausgenommen die therapeutische Behandlung des menschlichen oder tierischen Körpers oder human- oder veterinärmedizinische Diagnostizierverfahren, mit: Ankleben eines ggf. mehrere Wirkstoffe und/oder den gleichen Wirkstoff in unterschiedlichen oder gleichen Konzentrationen in verschiedenen, voneinander trennbaren Wirkstoffreservoirteilen aufweisenden Pflasters auf die Haut; Abziehen eines oder mehrerer Pflasterteile mit jeweils einem oder mehreren Wirstoffreservoirteilen nach einem vorherbestimmten Zeitraum unter Verbleiben eines Pflasterrestes mit mindestens einem Wirkstoffreservoirteil auf der Haut sowie ggf. Wiederholen des letzten Schrittes nach einem vorherbestimmten Zeitraum.

Nachfolgend wird die Erfindung anhand der Zeichnung näher erläutert, in deren Figuren bevorzugte Ausführungsformen der Erfindung schematisch und nicht maßstabsgetreu dargestellt sind. Dabei zeigt:

Fig.1    einen Querschnitt durch eine erste Ausführungsform eines erfindungsgemäßen Pflasters mit zweiteiligem Wirkstoffreservoir

Fig.2    eine Aufsicht auf die Hautseite einer zweiten Ausführungsform des erfindungsgemäßen Pflasters ohne Schutzschicht

Fig.3    den Querschnitt gemäß Linie I-I der Fig. 2

Fig.4    eine Aufsicht auf die Hautseite einer dritten erfindungsgemäßen Ausführungsform ohne Schutzschicht

Fig.5    einen Querschnitt durch die Ausführungsform der Fig.4 entlang Linie II-II in vergrößertem Maßstab

Fig.6    eine Aufsicht auf die Hautseite einer vierten Ausführungsform der Erfindung ohne Schutzschicht

Fig.7    einen Querschnitt durch die Ausführungsform der Fig. 6 entlang Linie III-III

Fig.8    einen Querschnitt durch eine fünfte Ausführungsform der Erfindung ;

Fig.9    einen Querschnitt durch eine sechste Ausführungsform der Erfindung

Fig.10   eine Aufsicht auf die Hautseite einer weiteren Ausführungsform eines erfindungsgemäßen Pflasters mit dreiteiligem Wirkstoffreservoir ohne Schutzschicht, und

Fig.11   einen vergrößerten Querschnitt entlang Linie IV-IV der Fig. 10

Im folgenden sollen die in den einzelnen Figuren dargestellten bevozugten Ausgestaltungen der Erfindung erläutert werden. Dabei wird der auf der Haut als letzter verbleibender Wirkstoffreservoirteil als erster Wirkstoffreservoirteil bezeichnet, das jeweils zweite und dritte Wirkstoffreservoirteil sind diejenigen, die mit der Rückschicht abgelöst werden.

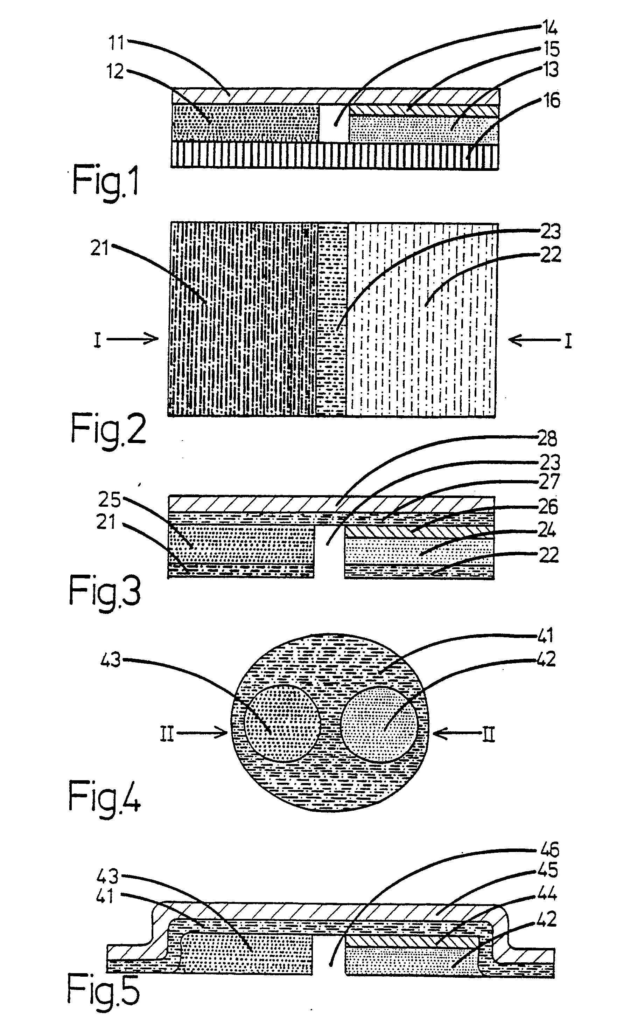

Fig. 1 zeigt eine bevorzugte Ausführungsform eines erfindungsgemäßen, ggf. runden oder eckigen Pflasters mit einem zweiteiligen haftklebenden Wirkstoffreservoir im Querschnitt. Der zweite Wirkstoffreservoirteil 12 haftet unmittelbar an der Rückschicht 11 und wird durch eine Reservoir-Trennschicht 14, die als Spalt/Zwischenraum zwischen den Wirkstoffreservoirteilen ausgebildet sein kann oder auch durch ein inertes Trennmaterial gefüllt

sein kann, vom ersten haftklebenden Wirkstoffreservoirteil 13 getrennt, das seinerseits über eine Ablöseschicht 15, die eine abgestufte Haftung der beiden Wirkstoffreservoirteile 12,13 an der Rückschicht verwirklicht, an der Rückschicht 11 haftet. Die Ablöseschicht 15 kann bspw. eine Polymer- oder Metallfolie, ein textiles Flächengebilde oder ein Laminat derselben sein und bildet nach Abziehen der Rückschicht 11 mit dem zweiten Wirkstoffreservoirteil 12 für den auf der Haut verbleibenden ersten Wirkstoffreservoirteil 13 eine Schutzschicht für den Schutz des verbleibenden ersten Wirkstoffreservoirteils 13. Dabei muß die Haftung des ersten Wirkstoff-reservoirteils 13 auf der Haut größer sein als die Haftung zwischen Ablöseschicht 15 und Rückschicht 11. Die Schutzschicht 16 wird vor Applikation des Pflasters entfernt. Dabei wird durch gleiche Punktierung der die Wirkstoffreservoirteile 12, 13 darstellenden Flächen in Fig.1 verdeutlicht, daß hier in beiden Wirkstoffreser-voirteilen 12,13 der gleiche Wirkstoff oder Wirkstoff-kombination vorliegt - mit dem Pflaster der Fig. 1 kann somit eine stufenförmige Abnahme der Wirkstoffabgabe an die Haut erzielt werden.

In Fig. 2 ist eine Aufsicht auf die Hautseite eines weiteren erfindungsgemäßen eckigen Pflasters mit zweiteiligem, nicht-haftklebendem Wirkstoffreservoir, dargestellt, wobei die Schutzschicht bereits abgelöst ist. Demzufolge besitzt das Pflaster hautseitige Haftklebeschichten 21,22 die auch durch die inerte Reservoir-Trennschicht 23, die wiederum ein einfacher Zwischenraum oder ein inertes Material sein kann, voneinander getrennt sind.

In Fig. 3 ist ein Querschnitt durch die in Fig. 2 dargestellte Ausführungsform entlang Linie I-I der Fig. 3 wiedergegeben. Die nichtklebenden Wirkstoffreservoirteile 24, 25 sind rückseitig mit einer Haftkleberschicht 27 an der Rückschicht 28 befestigt, wobei der erste Wirkstoffreservoirteil 24 über eine zwischen Haftkleberschicht 27 und erstem Wirkstoffreservoirteil 24 angeordnete Ablöseschicht 26 an der Rückschicht haftet. Die Ablöseschicht 26 ist dabei so ausgelegt, daß ihre Haftung am ersten Wirkstoffreservoirteil 24 größer ist als an der Haftkleberschicht 27. Damit verbleibt die Ablöseschicht 26 beim Ablösen der Rückschicht 28 und dem über die Haftkleberschicht 27 an der Rückschicht 28 befestigten zweiten Wirkstoffreservoirteil 25 gemeinsam mit dem ersten Wirkstoffreservoirteil 24 auf der Haut und übernimmt für diesen verbleibenden Wirkstoffreservoirteil 24 die Funktion einer Schutzschicht, die gegen Verunreinigung und Beschädigung des Reservoirmaterials von außen oder auch vor dem Entweichen ggf. flüchtiger Wirkstoffkomponenten schützt.

Die hautseitigen Haftklebeschichten 21 und 22 auf den nichtklebenden Wirkstoffreservoirteilen 24, 25 sind so eingestellt, daß die Haftung der Haftklebeschicht 22 des ersten Wirkstoffreservoirteils 24 auf der Haut größer ist als die Haftung zwischen Ablöseschicht 26 und Haftklebeschicht 27. Die unterschiedlich angelegten Flächen in der Zeichnung soll andeuten, daß in beiden Wirkstoffreservoirteilen 24,25 unterschiedliche Wirkstoffe oder Wirkstoffkombinationen vorliegen.

In Fig. 4 ist eine weitere bevorzugte Ausgestaltung eines erfindungsgemäßen Pflasters in Aufsicht auf die Hautseite desselben, dargestellt. Das ovale Pflaster besitzt ein zweiteiliges Wirkstoffreservoir,wobei eine Haftkleberschicht 41, die zur Fixierung der Wirkstoffreservoirteile 42,43 an der Rückschicht 45 sorgt, auch die Befestigung des therapeutischen Systems an der Haut bildet.

In Fig. 5 ist ein vergrößerter Querschnitt nach Linie II-II in Fig. 4 dargestellt. Die haftklebenden Wirkstoffreservoirteile 42 und 43, die durch eine hier als Zwischenraum ausgebildete inerte Reservoir-Trennschicht 46 voneinander getrennt werden, sind beutelartig von der mit einer Haftklebeschicht 41 belegten Rückschicht 45 umgeben. Auch hier sorgt eine Trennschicht 44 dafür, daß die Haftung des ersten Wirkstoffreservoirteils 42 auf der Haut größer ist als die Haftung zwischen der Ablöseschicht 44 und der Haftklebeschicht 41 und dafür, daß der auf der Haut nach Ablösen des zweiten Wirkstoffreservoirteils 43 und der Rückschicht 45 verbleibende erste Wirkstoffreservoirteil 42 durch eine Schicht geschützt ist. Die beiden Wirkstoffreservoirteile 42 und 43 beinhalten, wie es durch die Anlage der Flächen verdeutlicht wird, unterschiedliche Wirkstoffe oder Wirkstoffkombinationen.

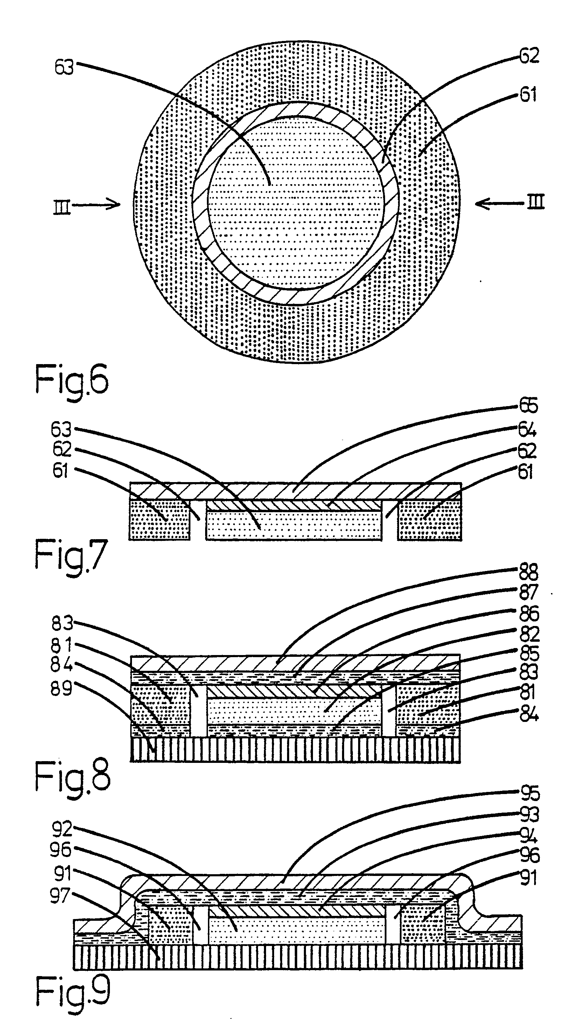

In Fig. 6 ist ein weiteres erfindungsgemäßes Pflaster mit zweiteiligem Wirkstoffreservoir, bei dem ein Wirkstoffreservoirteil 61 den anderen Wirkstoffreservoirteil 63 konzentrisch umschließt, von der Hautseite her, bei abgezogener Schutzschicht, dargestellt. Das haftklebende erste kreisförmige Wirkstoffreservoir 63 ist vom ringförmigen zweiten Wirkstoffreservoir 61, getrennt durch eine inerte Reservoir-Trennschicht 62, die auch als Zwischenraum ausgebildet sein kann, umgeben. Diese Ausführungsform hat den Vorteil, daß bei Reduktion der Wirkstoffabgabefläche durch Entfernen eines Teils des Pflasters mit dem zweiten Wirkstoffreservoirteil 61 gemeinsam mit der Rückschicht 65 die Abzugsrichtung des Pflasters frei wählbar ist.

In Fig. 7 ist der Querschnitt durch die in Fig. 6 dargestellte Ausführungsform entlang der Linie III-III der Fig. 6, dargestellt, Der ringförmige zweite Wirkstoffreservoirteil 61 grenzt unmittelbar an die Rückschicht 65, während die Ablöseschicht 64 zwischen dem kreisförmigen ersten Wirkstoffreservoirteil 63 und der Rückschicht 65 angeordnet ist. Die Haftung des ersten Wirkstoffreservoirteils 63 auf der Haut und diejenige zwischen Ablöseschicht 64 und Rückschicht 65 ist größer als die Haftung zwischen Ablöseschicht 64 und Wirkstoffreservoirteil 63, so daß beim Abziehen der Rückschicht 65 der zweite Wirkstoffreservoirteil 61 unter Verbleiben des ersten Wirkstoffreservoirteils 63 an der Haut entfernt wird, der nun von der Ablöseschicht 64 bedeckt wird. Die inerte Reservoirtrennschicht 62 trennt die Wirkstoffreservoirteile 61 und 63. Bei dieser Ausführungsform ist der Wirkstoff in beiden Wirkstoffreservoirteilen 61, 63 der gleiche.

In Fig. 8 ist eine weitere bevorzugte Ausführungsform der Erfindung im Querschnitt dargestellt, bei der die beiden Teile eines nichtklebenden Wirkstoffreservoirs wieder als Kreisscheibe 82 mit umgebendem Ring 81 ausgebildet sind. Sie sind durch eine inerte Reservoir-Trennschicht 83 voneinander getrennt. Die Rückschicht 88 ist mit einer Haftkleberschicht 87 belegt, die mit dem zweiten Wirkstoffreservoirteil 81 direkt in Kontakt steht.

Zwischen der Haftkleberschicht 87 und dem ersten Wirkstoffreservoirteil 82 befindet sich eine inerte Ablöseschicht 86, die derart ausgelegt ist, daß ihre Haftung zur Haftkleberschicht 87 geringer als die zwischen der Haftkleberschicht 85 und der Haut ist. Die übrigen Haftwerte an den Grenzschichten dieses Pflasterteils müssen natürlich oberhalb des Haftwertes an der Haut liegen. Die hautseitigen Haftklebeschichten 84 und 85 der Wirkstoffreservoirteile 81,82 stellen den Kontakt mit der Haut sicher und sind vor Applikation des erfindungsgemäßen Pflasters vorteilhafterweise durch eine Schutzschicht 89 abgedeckt.

Bei dieser Ausführungsform weisen beide Wirkstoffreservoirteile 81,82 unterschiedliche Wirkstoffe oder Wirkstoffkombinationen auf.

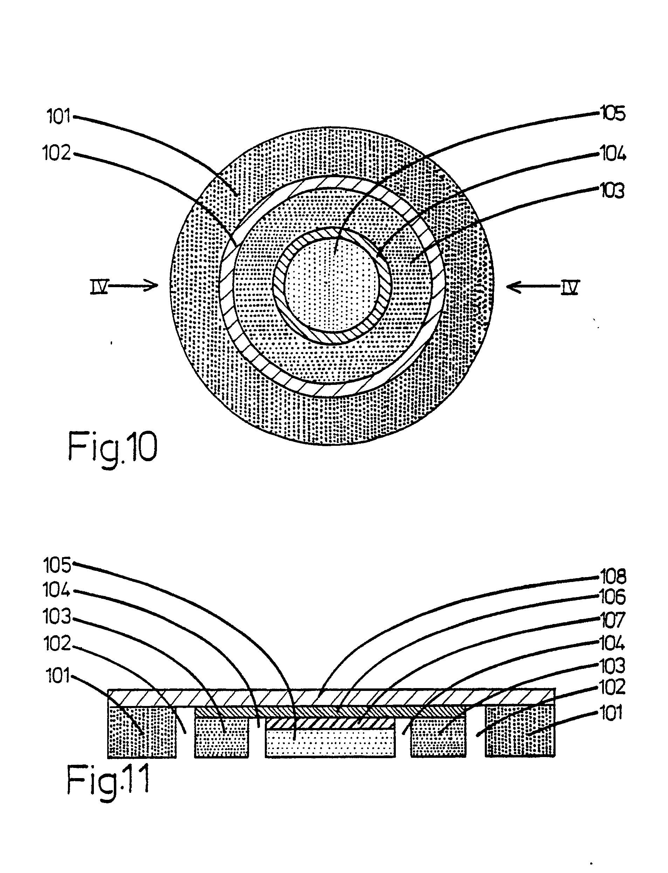

In Fig. 9 ist ein Querschnitt durch eine weitere Ausführungsform der Erfindung dargestellt, wobei die Rückschicht 95 die konzentrische Anordung der haftklebenden Wirkstoffreservoirteile 91 und 92 bis auf die Hautseite umschließt. Zur Befestigung des Gesamtpflasters und der Wirkstoffreservoirteile an der Haut ist die Rückschicht 95 vollflächig mit einer Haftklebeschicht 93 versehen. An dieser klebt der zweite Wirkstoffreservoirteil 91 direkt, während der erste Wirkstoffreservoirteil 92 über eine Ablöseschicht 94 befestigt ist. Die Haftung der Ablöseschicht 94 zum Wirkstoffreservoirteil 92 liegt über und die Haftung der Ablöseschicht 94 zur Haftklebeschicht 93 liegt unter der Haftung zwischen dem ersten Wirkstoffreservoirteil 92 und der Haut. So wird ein selektives Abziehen der Rückschicht 95 gemeinsam mit dem zweiten Wirkstoffreservoirteil 91 ermöglicht. Natürlich kann durch entsprechende Änderung des Aufbaus auch ein selektives Abziehen des ersten Wirkstoffreservoirteils 92 realisiert werden. Mit dem Bezugzeichen 96 ist die inerte Reservoir-Trennschicht oder der Zwischenraum zwischen den Wirkstoffreservoirteilen 91 und 92 bezeichnet. Die gesamte Anordnung ist bis zur Applikation von einer ablösbaren Schutzschicht 97 abgedeckt. Die Wirkstoffe oder Wirkstoffkombinationen sind in dieser Ausführungsform in beiden Wirkstoffreservoirteilen 91,92 unterschiedlich.

Eine weitere bevorzugte Ausführungsform der Erfindung mit dreigeteiltem Wirkstoffreservoir ist in Fig.10 gegeben. Sie zeigt eine Aufsicht auf die Hautseite eines erfindungsgemäßen Pflasters ohne Schutzschicht. Ein erstes kreisförmiges haftklebendes Wirkstoffreservoirteil 105 wird von einem zweiten ringförmigen haftklebenden Wirkstoffreservoirteil 103, von diesem durch eine inerte Reservoir-Trennschicht 104 getrennt, umgeben, das dritte haftklebende Wirkstoffreservoirteil 101 umschließt das zweite Wirkstoffreservoirteil 103 in konzentrischer Ringform; wobei zwischen dem zweiten und dritten Wirkstoffreservoirteil 101, 103 eine weitere inerte Reservoir-Trennschicht 102 vorgesehen ist; einen vergrößerten Querschnitt nach Linie IV-IV gibt die Fig. 11 wieder.

Dabei klebt der Wirkstoffreservoirteil 101 unmittelbar an der Rückschicht 108. Eine mit der Rückschicht 108 verbundene erste Ablöseschicht 106 deckt die Wirkstoffreservoirteile 103 und 105 ab. Ihre Haftung an der Rückschicht 108 ist geringer als die Haftung der Wirkstoffreservoirteile 103 und 105 an der Haut, so daß mit Abziehen der Rückschicht 108 mit Wirkstoffreservoirteil 101 die Wirkstoffreservoirteile 103 und 105 auf der Haut verbleiben. Die erste Ablöseschicht 106 bildet sodann eine schützende neue Schicht für die verbleibenden Teile des Pflasters. Der haftklebende Wirkstoffreservoirteil 103 klebt unmittelbar an der ersten Ablöseschicht 106 und kann gemeinsam mit dieser in einem weiteren Ablösungsschritt unter Hinterlassen des ersten Wirkstoffreservoirteils 105 auf der Haut entfernt werden, was durch die zweite Ablöseschicht 107 zwischen dem ersten Wirkstoffreservoirteil 105 und der ersten Ablöseschicht 106 ermöglicht wird. Die Haftung der zweiten Ablöseschicht 107 an der ersten 106 ist geringer als die Haftung des ersten Wirkstoffreservoirteils 105 an der Haut. Die inerten Reservoir-Trennschichten 102 und 104, die auch als Zwischenräume ausgebildet sein können, liegen zwischen den Wirkstoffreservoirteilen 101 und 103 bzw. 102 und 105. Das ausgewählte Beispiel zeigt für die Wirkstoffreservoirteile 103 und 105 denselben Wirkstoff oder derselbe Wirkstoffkombination, wie durch die gleiche Punktierung in der Fig. 11 angedeutet ist, während der Wirkstoffreservoirteil 101 einen davon verschiedenen Wirkstoff bzw. Wirkstoffkombination aufweist.

Die lediglich beispielhaft die Erfindung erläuternden Figuren sollen die Erfindung weder hinsichtlich ihrer geometrischen Form, der Zuordnung bestimmter Einzelkomponenten zueinander noch hinsichtlich der Größe der Wirkstoffabgabeflächen einschränken; alle diese Größen können, wie es dem Fachmann auf diesem Gebiet geläufig ist, entsprechend den therapeutischen Erfordernissen angepaßt sein, wobei selbstverständlich die Gesichtspunkte rationeller Fertigung zu berücksichtigen sind. Auch der abzulösende Teil des Wirkstoffreservoirteils kann eine Ablöseschicht aufweisen, die dann eine größere Haftung zur Rückschicht aufweist als die Ablöseschicht des auf der Haut verbleibenden Reservoirteils. Diese Konstruktion ist dann von Vorteil, wenn die Wirkstoffreservoirteile die gleiche Dicke haben sollen. Die Haftklebeschichten, insbesondere die hautseitigen, können zur verbesserten Wirkstoffdurchlässigkeit haftkleberfreie Bereiche aufweisen oder nur als vereinzelte Haftklebeflächen, bspw. im Wirkstoffreservoirmaterial eingebettet, ausgebildet sein. Die Freisetzung der Wirkstoffe aus den Wirkstoffreservoirteilen kann ggf. auch durch Steuermembranen geregelt werden, die in einem oder mehreren Wirkstoffreservoirteile(n) in an sich bekannter Weise in die Reservoirmasse eingebettet sind oder sich zwischen Wirkstoffreservoir und der hautseitigen Haftklebeschicht befinden oder auch innerhalb der hautseitigen Haftklebeschicht.

Für den Aufbau eines Wirkstoffreservoirs, wie es erfindungsgemäß eingesetzt werden kann, eignen sich die dem Fachmann geläufigen Maßnahmen und Materialien; als Basismaterialien können nieder- und hochmolekulare natürliche und/oder synthetische Stoffe dienen, deren Auswahl sich nach den Eigenschaften der zu verabreichenden Wirkstoffe und den therapeutischen Erfordernissen richtet. Das Wirkstoffreservoir kann neben dem Basismaterial auch weitere geeignete, dem Fachmann aufgrund seines Fachwissens bekannte bzw. naheliegende Zusätze, wie bspw. Lösungsvermittler, Weichmacher, Klebrigmacher, Stabilisatoren, Füllstoffe und Enhancer beinhalten. Die Zusammensetzung kann in den Reservoirteilen gleich oder verschieden sein, was wiederum von den zu verabreichenden Wirkstoffen und den gewünschten Freisetzungsraten bzw. der Kinetik abhängt.

Bei gleicher Reservoirzusammensetzung und nur einem zu verabreichenden Wirkstoff kann eine abgestufte Konzentrationseinstellung des Wirkstoffes in den verschiedenen Reservoirteilen angezeigt sein. Als Wirkstoffe für das erfindungsgemäße Pflaster sind im Prinzip alle geeignet, die alleine oder mit Hilfsmitteln in die Haut zu wandern vermögen.

Die das Wirkstoffreservoir an der hautabgewandten Seite abdeckende Rückschicht kann durchlässig oder undurchlässig sein. Sie muß flexibel sein und dient wegen der mechanischen Stabilisierung des Pflasters zum Abziehen eines Teils des Wirkstoffreservoirs. Sind Bestandteile der Reservoirs oder eingearbeitete Wirkstoffe flüchtig, so muß die Rückschicht für diese Stoffe undurchlässig sein. Sie kann ein- oder mehrschichtig sein. Materialien, die zu ihrer Herstellung geeignet sind, umfassen beispielsweise polymere Substanzen, wie Polyethylen, Polypropylen, Polyester und Polyamide. Als weitere Materialien können auch Metallfolien, wie bspw. Aluminiumfolie, alleine oder mit einem polymeren Substrat beschichtet, eingesetzt werden.

Durchlässige Rückschichten sind bspw. textile Flächengebilde, wie Gelege und Vliesstoffe, oder auch poröse Polymermaterialien. Die Rückschicht kann ggf. eine Markierung tragen, um die optimale Abziehrichtung des Pflasters zu kennzeichnen. Gegebenenfalls sind zwischen den Wirkstoffreservoirteilen anstelle eines Zwischenraums Ablöseschichten vorgesehen. Für sie sind die gleichen Materialien geeignet, wie sie weiter oben für die Trennschichten zwischen Wirkstoffreservoirteilen und Rückschicht beschrieben sind. Die vor der Applikation ablösbare Schutzschicht, die die hautseitigen Haftklebeflächen abdeckt, kann aus den gleichen Materialien, wie sie zur Herstellung der Rückschicht eingesetzt werden, hergestellt sein, vorausgesetzt, daß sie ablösbar gemacht werden, bspw. durch Aufbringen einer Siliconschicht. Andere ablösbare Schutzschichten, wie sie dem Fachmann auf dem in Rede stehenden Gebiet der Pflaster und insbesondere pflasterartigen therapeutischen Systeme bekannt sind, sind bspw. Polytetrafluorethylen, behandeltes Papier, Zellophan, Polyvinylchlorid u.ä. Zur leichteren Ablösbarkeit kann die Schutzschicht in an sich bekannter Weise mit Abziehhilfen versehen sein. Die Schutzschicht kann auch größer sein als das Pflaster, bspw. dann, wenn mehrere Pflaster auf einer ununterbrochenen Bahn des Schutzschichtmaterials angeordnet sind.

Für die im erfindungsgemäßen Pflaster eingesetzten Haftkleberschichten sind alle physiologisch unbedenklichen, gegenüber Wirkstoffen und übrigen Wirkstoffreservoirbestandteilen inerten Haftkleber geeignet, bspw. auf Basis von Kautschuk, kautschukähnlichen synthetischen Homo-, Co- oder Blockpolymeren, Polyacrylsäureestern und deren Copolymerisaten, Polyurethanen und Silikonen.

Zur Herstellung der erfindungsgemäßen Pflaster kann auf die dem Fachmann bekannten Verfahren der Pflastertechnologie zurückgegriffen werden. Dies wird an dem nachfolgenden Herstellungsbeispiel für ein Pflaster mit zweiteiligem Wirkstoffreservoir demonstriert.

Herstellungsbeispiel

Im Walzenauftragsverfahren wird auf eine durch Siliconisierung abweisend ausgrüstete Folie, die als Zwischenabdeckung dient, eine wirkstoffhaltige Reservoirmaterial-Lösung aufgebracht, die nach der Trocknung bei 65 °C eine haftklebende Schicht von 55 g/m$^2$ bildet. Diese Schicht setzt sich, wie folgt, zusammen:

1. Acrylatcopolymer I 68,86 Gew.-Teile
2. Acrylatcopolymer II 10,39 Gew.-Teile
3. POÄ-(10)-Oleylalkohol 5,20 Gew.-Teile
4. Wirkstoff 15,56 Gew.-Teile

Dabei bedeuten:

Acrylatcopolymer I:
aus Durotac 280-2516 der Fa.National Stach & Chemical B.V., Niederlande
Acrylatcopolymer II:
aus Eudragit E100 der Fa.Röhm Pharma, Deutschland
POÄ-(10)-Oleylalkohol:
Brij 97 der Fa. Atlas Chemical Industries, GB

Zur einen Hälfte der wirkstoffhaltigen Haftkleberschicht wird eine nicht siliconisierte Polyesterfolie (Dicke 0,036 mm) und zur anderen Hälfte die nicht silikonisierte Seite einer einseitig siliconisierten Polyester-Folie (Dicke 0.036 mm) kaschiert. Nach Abziehen der Zwischenabdeckung werden aus der ersten Hälfte die ringförmigen Wirkstoffreservoirteile herausgestanzt, die mit der haftklebenden Seite auf eine siliconisierte aluminiumbedampfte Polyester-Folie (Schutzschicht) aufgelegt werden. Aus der zweiten Hälfte werden scheibchenförmige Wirkstoffreservoirteile ausgestanzt, die im Durchmesser geringfügig kleiner sind als der Innendurchmesser der Ringe, in die sie mit der haftklebenden Seite zur Schutzschicht gewandt zentrisch eingesetzt werden. Die offene Seite des so erhaltenen Verbundes wird mit einer haftklebend ausgerüsteten (Durotac 280-2516, 30 g/m$^2$ trocken) Rückschicht abgedeckt.

**Patentansprüche**

1. Wirkstoffhaltiges Pflaster zur kontrollierten Verabreichung von Wirkstoffen an die Haut, das eine Rückseite und eine Hautseite besitzt, mit einer Rückschicht (11, 28, 45, 65, 88, 95, 108), einem im wesentlichen senkrecht zur Hautkontaktfläche des Pflasters geteilten, einen oder mehrere Wirkstoffe aufweisenden Wirkstoffreservoir (12, 25, 43, 61, 81, 91, 101, 103, 13, 24, 42, 63, 82, 92, 105), einer Haftklebe-einrichtung auf der Hautseite sowie ggf. einer vor Applikation des Pflasters ablösbaren Abdeckschicht (16, 89, 97, 106), wobei die Wirkstoffabgabefläche des Pflasters um eine vorherbestimmte Fläche durch Entfernung eines Teils des Wirkstoffreservoirs verkleinerbar ist, dadurch gekennzeichnet, daß mindestens ein Wirkstoffreservoirteil (12, 25, 43, 61, 81, 91, 101, 103) unter Verbleiben eines oder mehrerer Wirkstoffreservoirteile (13, 24, 42, 63, 82, 92, 105) auf der Haut ablösbar ist, wobei der auf der Haut verbleibende Teil des Wirkstoffreservoirteil eine bessere Haftung an der Haut als an der Rückschicht (11, 28, 45, 65, 88, 95, 108) aufweist.

2. Wirkstoffhaltiges Pflaster nach Anspruch 1, dadurch gekennzeichnet, daß das Wirkstoffreservoir zweiteilig ist.

3. Wirkstoffhaltiges Pflaster nach Anspruch 1, dadurch gekennzeichnet, daß das Wirkstoffreservoir dreiteilig ist.

4. Wirkstoffhaltiges Pflaster nach einem der vorangehenden Ansprüche, dadurch gekennzeichnet, daß die Wirkstoffabgabeflächen der Wirkstoffreservoirteile geometrisch gleich oder unterschiedlich sind.

5. Wirkstoffhaltiges Pflaster nach einem der vorangehenden Ansprüche, dadurch gekennzeichnet, daß ein Wirkstoffreservoirteil (61, 81, 91, 101, 103) ein anderes oder mehrere andere Wirkstoffreservoirteil(e) (63, 82, 92, 103, 105) flächenmäßig betrachtet, vollständig umschließt.

6. Wirkstoffhaltiges Pflaster nach Anspruch 4 oder 5, dadurch gekennzeichnet, daß ein Wirkstoffreservoirteil (61, 81, 91, 101, 103) ringförmig einen oder mehrere andere Wirkstoffreservoirteil(e) (63, 82, 92, 103, 105) umschließt.

7. Wirkstoffhaltiges Pflaster nach einem der Ansprüche 1 bis 5, dadurch gekennzeichnet, daß das Abgabeflächen-Größenverhältnis eines Wirkstoffreservoirteils zu einem anderen zwischen etwa 1:1 bis 1:10 beträgt.

8. Wirkstoffhaltiges Pflaster nach einem der vorangehenden Ansprüche, dadurch gekennzeichnet, daß in allen Wirkstoffreservoirteilen derselbe Wirkstoff oder dieselbe Wirkstoffkombination vorliegt.

9. Wirkstoffhaltiges Pflaster nach einem der Ansprüche 1 bis 7, dadurch gekennzeichnet, daß in mindestens einem Wirkstoffreservoirteil ein(e) andere(r) Wirkstoff/ Wirkstoffkombination als in dem anderen Wirkstoffreservoirteil vorliegt.

10. Wirkstoffhaltiges Pflaster nach einem der vorangehenden Ansprüche, dadurch gekennzeichnet, daß die Wirkstoffe eine topische oder systemische Wirkung besitzen.

11. Verfahren zur kontrollierten Verabreichung von einem oder mehreren Wirkstoffen an die Haut mittels eines Pflasters gemäß einem der vorangehenden Ansprüche, jedoch ausgenommen die therapeutische Behandlung des menschlichen oder tierischen Körpers oder human- oder veterinärmedizinische Diagnostizierverfahren, gekennzeichnet durch: Ankleben eines ggf. mehrere Wirkstoffe und/oder den gleichen Wirkstoff in unterschiedlichen oder gleichen Konzentrationen in verschiedenen, voneinander trennbaren Wirkstoffreservoirteilen aufweisenden Pflasters auf die Haut; Abziehen eines oder mehrerer Pflasterteile mit jeweils einem oder mehreren Wirstoffreservoirteilen nach einem vorherbestimmten Zeitraum unter Verbleiben eines Pflasterrestes mit mindestens einem Wirkstoffreservoirteil auf der Haut sowie ggf. Wiederholen des letzten Schrittes nach einem vorherbestimmten Zeitraum.

12. Verwendung des wirkstoffhaltigen Pflasters nach einem der vorangehenden Ansprüche für die lokale und/oder systemische transdermale Wirkstoffverabreichung in der Kosmetik.

**Claims**

1. Active substance-containing plaster for the controlled administration of active substances onto the skin, which has a backing side and a skin side, with a backing layer (11, 28, 45, 65, 88, 95, 108), with an active substance reservoir (12, 25, 43, 61, 81, 91, 101, 103, 13, 24, 42, 63, 82, 92, 105) which is divided essentially at right angles to the skin contact area of the plaster and has one or more active substances, with an adhesive device on the skin side, and, where appropriate, with a covering layer (16, 89, 97, 106) which can be detached before application of the plaster, where the active substance delivery area of the plaster can be reduced in size by a predetermined area by removing a part of the active substance reservoir, characterised in that at least one active substance reservoir part (12, 25, 43, 61, 81, 91, 101, 103) can be detached while leaving one or more active substance reservoir parts (13, 24, 42, 63, 82, 92, 105) on the skin, where the part of the active substance reservoir part remaining on the skin has better adhesion to the skin than to the backing layer (11, 28, 45, 65, 88, 95, 108).

2. Active substance-containing plaster according to Claim 1, characterised in that the active substance reservoir is in two parts.

3. Active substance-containing plaster according to Claim 1, characterised in that the active substance reservoir is in three parts.

4. Active substance-containing plaster according to any of the preceding claims, characterised in that the active substance delivery areas of the active substance reservoir parts are geometrically identical or different.

5. Active substance-containing plaster according to any of the preceding claims, characterised in that an active substance reservoir part (61, 81, 91, 101, 103) completely surrounds one other or several other active substance reservoir part(s) (63, 82, 92, 103, 105) viewed in terms of surface.

6. Active substance-containing plaster according to Claim 4 or 5, characterised in that an active substance reservoir part (61, 81, 91, 101, 103) surrounds in the form of a ring one or more other active substance reservoir part(s) (63, 82, 92, 103, 105).

7. Active substance-containing plaster according to any of Claims 1 to 5, characterised in that the delivery area/size ratio of one active substance reservoir part to another is between about 1:1 to 1:10.

8. Active substance-containing plaster according to any of the preceding claims, characterised in that the same active substance or the same active substance combination is present in all active substance reservoir parts.

9. Active substance-containing plaster according to any of Claims 1 to 7, characterised in that a different active substance/active substance combination is present in at least one active substance reservoir part than in the other active substance reservoir part.

10. Active substance-containing plaster according to any of the preceding claims, characterised in that the active substances have a topical or systemic action.

11. Method for the controlled administration of one or more active substances onto the skin by means of a plaster according to any of the preceding claims, but excepting the therapeutic treatment of the human or animal body or diagnostic methods in human or veterinary medicine, characterised by: adhesion onto the skin of a plaster which, where appropriate, has a plurality of active substances and/or the same active substance in different or identical concentrations in different active substance reservoir parts which can be separated from one another; pulling off of one or more plaster parts with in each case one or more active substance reservoir parts after a predetermined period with retention of a plaster residue with at least one active substance reservoir part on the skin, and, where appropriate, repetition of the last step after a predetermined period.

12. Use of the active substance-containing plaster according to any of the preceding claims for local and/or systemic transdermal active substance administration in cosmetics.

**Revendications**

1. Bandage à principe(s) actif(s) pour contrôler l'administration de principes actifs sur la peau, qui possède un côté postérieur et un côté peau, comportant une couche postérieure (11, 28, 45, 65, 88, 95, 108), un réservoir de principe(s) actif(s) (12, 25, 43, 61, 81, 91, 101, 103, 13, 24, 42, 63, 82, 92, 105) comprenant un ou plusieurs principes actifs répartis en substance perpendiculairement à la surface de contact avec la peau du bandage, un dispositif autocollant sur le côté peau ainsi que, éventuellement, une couche de recouvrement (16, 89, 97, 106) détachable avant l'application du bandage, la surface du bandage libérant le principe actif pouvant être réduite d'une fraction de surface prédéterminée par élimination d'une partie du réservoir de principe(s) actif(s), le bandage étant caractérisé en ce qu'au moins une partie du réservoir de principe(s) actif(s) (12, 25, 43, 61, 81, 91, 101, 103) est détachable en laissant subsister une ou plusieurs parties du réservoir de principe(s) actif(s) (13, 24, 41, 63, 82, 92, 105), la partie du réservoir de principe(s) actif(s) subsistant sur la peau présentant une meilleure adhérence à la peau que sur la couche postérieure (11, 28, 45, 65, 88, 95, 108).

2. Bandage à principe(s) actif(s) selon la revendication 1, caractérisé en ce que le réservoir de principe(s) actif(s) comporte deux parties.

3. Bandage à principe actif selon la revendication 1, caractérisé en ce que le réservoir de principe(s) actif(s) comporte trois parties.

4. Bandage à principe(s) actif(s) selon l'une quelconque des revendications précédentes, caractérisé en ce que les surfaces de libération des principes actifs des parties de réservoir de principe(s) actif(s) sont géométriquement égales ou différentes.

5. Bandage à principe(s) actif(s) selon l'une quelconque des revendications précédentes, caractérisé en ce qu'une partie du réservoir de principe(s) actif(s) (61, 81, 91, 101, 103) entoure complètement une autre ou plusieurs autres parties du réservoir de principe(s) actif(s) (63, 82, 92, 103, 105) sur le plan superficiel.

6. Bandage à principe(s) actif(s) selon la revendication 4 ou 5, caractérisé en ce qu'une partie du réservoir de principe(s) actif(s) (61, 81, 91, 101, 103) entoure une ou plusieurs autres parties du réservoir de principe(s) actif(s) (63, 82, 92, 103, 105) de façon annulaire.

7. Bandage à principe(s) actif(s) selon l'une quelconque des revendications 1 à 5, caractérisé en ce que le rapport dimensionnel des surfaces de libération d'une partie du réservoir de principe(s) actif(s) vis-à-vis d'une autre se situe entre environ 1:1 et 1:10.

8. Bandage à principe(s) actif(s) selon l'une quelconque des revendications précédentes, caractérisé en ce que, dans toutes les parties de réservoir de principe(s) actif(s), il y a le même principe actif ou la même combinaison de principes actifs.

9. Bandage à principe(s) actif(s) selon l'une quelconque des revendications 1 à 7, caractérisé en ce qu'il y a dans au moins une partie du réservoir de principe(s) actif(s) un autre principe actif ou une autre combinaison de principes actifs différent(s) de ceux de l'autre partie de réservoir de principe(s) actif(s).

10. Bandage à principe(s) actif(s) selon l'une quelconque des revendications précédentes, caractérisé en ce que les principes actifs possèdent une action topique ou systémique.

11. Procédé de contrôle de l'administration d'un ou plusieurs principes actifs sur la peau à l'aide d'un bandage selon l'une quelconque des revendications précédentes, mais en dehors du cadre du traitement thérapeutique du corps humain ou animal ou du processus de diagnostic médical humain ou vétérinaire, caractérisé en ce qu'on applique un ou plusieurs principes actifs et/ou le même principe actif en concentrations différentes ou égales sur un bandage présentant diverses parties de réservoirs de principe(s) actif(s) séparables l'une de l'autre sur la peau, on retire une ou plusieurs parties du bandage avec respectivement une ou plusieurs parties de réservoirs de principe(s) actif(s) après un temps prédéterminé en laissant subsister un reste de bandage avec au moins une partie de réservoir de principe(s) actif(s) sur la peau et éventuellement, on répète la dernière étape après un intervalle de temps prédéterminé.

12. Emploi du bandage à principe(s) actif(s) selon l'une quelconque des revendications précédentes pour l'administration transdermique locale et/ou systémique d'un principe actif en cosmétique.

Fig.1

Fig.2

Fig.3

Fig.4

Fig.5

EP 0 288 734 B1

Fig.6

Fig.7

Fig.8

Fig.9

12

101 —
102 —

105
104
103

IV →

← IV

# Fig.10

105
104
103
102
101

108
106
107
104
103
102
101

# Fig.11

13